(19) Europäisches Patentamt
European Patent Office
Office européen des brevets

(11) Veröffentlichungsnummer: **0 039 895**
**B1**

(12) # EUROPÄISCHE PATENTSCHRIFT

(45) Veröffentlichungstag der Patentschrift: **18.07.84**

(51) Int. Cl.³: **C 07 J 9/00**, C 07 J 13/00, C 07 J 41/00

(21) Anmeldenummer: **81103424.8**

(22) Anmeldetag: **06.05.81**

(54) **Neue Pregnan-20-Carbonsäurederivate und Verfahren zu ihrer Herstellung.**

(30) Priorität: **12.05.80 AT 2535/80**

(43) Veröffentlichungstag der Anmeldung: **18.11.81 Patentblatt 81/46**

(45) Bekanntmachung des Hinweises auf die Patenterteilung: **18.07.84 Patentblatt 84/29**

(84) Benannte Vertragsstaaten: **BE CH FR LI NL**

(56) Entgegenhaltungen:
**DE - A - 2 839 033**
**US - A - 2 492 188**
**US - A - 2 752 369**
**US - A - 4 088 760**
**US - A - 4 255 345**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Preuss, Wolfgang, Dr., Finkenweg 12, D-4019 Monheim (DE)**

**EP 0 039 895 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die offengelegte Europäische Patentanmeldung 79 101 036.6 (004 913) schildert unter anderem ein Verfahren zur Herstellung von 17-C-Steroid-$\alpha$-Propionsäureverbindungen — insbesondere zur Herstellung von 3-Oxo-pregna-4-en-20-carbonsäure ($\Delta$4-BNC) und/oder 3-Oxo-pregna-1,4-dien-20-carbonsäure ($\Delta$1,4-BNC) — durch mikrobiellen Seitenkettenabbau an 17-C-Seitenketten-Steroidsubstraten. Durch Verwendung in bestimmter Weise gezüchteter und ausgewählter Mikroorganismen-Defektmutanten, die auch in Abwesenheit von den Steroidringabbau und/oder das Wachstum hemmenden Inhibitoren Steroidverbindungen mit dem 17-C-$\alpha$-Propionsäurerest liefern, gelingt die Gewinnung von $\Delta$-4-BNC und insbesondere $\Delta$1,4-BNC in technischen Mengen. Eine weitere Ausgestaltung dieses Verfahrens ist in der Europäischen Patentanmeldung 79 104 165.0 (EP-A-0 015 308) beschrieben.

In der Europäischen Patentanmeldung 81 100 146.0 (EP-A-33 439) wird die Gewinnung von 20 Carboxy-pregna-1,4,17(20)-trien-3-on ($\Delta$1,4,17-BNC) durch mikrobiellen Seitenkettenabbau an 17-C-Seitenketten-Steroidsubstraten beschrieben. Die Umwandlung der 20-Carbonsäuregruppe in den entsprechenden Ester bzw. das Carbonsäurechlorid ist in der Europäischen Patentanmeldung 81 100 145.2 (EP-A-34 248) beschrieben.

Die US-PS 3 994 933 beschreibt die Herstellung von 3-Oxo-pregna-4,17(20)-dien-20-carbonsäure ($\Delta$4,17-BNC), ihren niederen Alkylestern und pharmakologisch verträglichen Salzen. Die Säure wird auch hier durch mikrobiellen Seitenkettenabbau von 17-C-Steroidverbindungen gewonnen.

Diese durch Seitenkettenabbau von Sterinen gebildeten BNC-Verbindungen ($\Delta$4-BNC, $\Delta$1,4-BNC, $\Delta$1,4,17-BNC und $\Delta$4,17-BNC) tragen nur in Position 3 eine funktionelle Gruppe im Ringsystem. Alle pharmakologisch wirksamen Corticosteroide enthalten jedoch zusätzliche Sauerstoff-Funktionen. Besonders wichtig sind dabei die Positionen 11, 17 und 21. Üblicherweise werden einige dieser Sauerstoff-Funktionen chemisch eingeführt, dazu gehören insbesondere die Positionen 17 und 21.

Die Oxidation der Position 11 in Steroidverbindungen erfolgt dagegen bevorzugt mikrobiell. In der Fachliteratur ist eine Vielzahl mikrobieller Steroidoxidationen in Position 11 beschrieben. Verwiesen wird in diesem Zusammenhang auf die folgenden Literaturreferate sowie die dort genannten Originalveröffentlichungen:

F. Drawert »Biosynthese von Hydroxy-Verbindungen«; Houben Weyl »Methoden der organischen Chemie« (1978) 6/1d, Seiten 378 bis 388;
T. H. Stoudt, Adv. Appl. Mikrobiol. 2 (1960), Seiten 190 bis 195, sowie
Handbuch W. Charney und H. L. Herzog »Mikrobial Transformations of Steroids« Academic Press (1967) New York, Seite 29.

Beschrieben wird hier mit zahlreichen Verweisungen auf bestimmte Mikroorganismenstämme, insbesondere aus der Klasse der Fungi, die mikrobielle 11-Hydroxylierung verschiedenartiger Steroidverbindungen und die dabei entstehenden Syntheseprodukte.

Für eine hohe pharmakologische Wirksamkeit ist im allgemeinen die Konfiguration 11$\beta$-Hydroxyl oder 11-Oxo erwünscht. Um in Position 11$\beta$-hydroxylierte Steroide zu erhalten, werden entweder Mikroorganismenstämme verwendet, die eine derartige Hydroxylgruppe stereoselektiv einführen, oder es werden andere Mikroorganismen verwendet, die überwiegend oder ausschließlich stereoselektiv in 11$\alpha$-Position hydroxylieren. In diesem Fall wird zur Gewinnung der 11$\beta$-hydroxylierten Steroide zunächst chemisch zum 11-Keton oxidiert und anschließend mit einem geeigneten Reduktionsmittel reduziert, wobei stereoselektiv die 11$\beta$-Hydroxyverbindung gebildet werden kann. Zur einschlägigen Literatur dieser anschließenden chemischen Umwandlung wird beispielsweise auf L. F. Fieser, M. Fieser »Steroide«, Verlag Chemie (Weinheim 1961) Seiten 73 ff sowie die dort zitierten Originalliteraturstellen J. Am. Chem. Soc. 77, 4436 (1955) verwiesen.

BNC-Verbindungen mit einer Sauerstoff-Funktion in 11-Position werden in der DE-OS 2 839 033 beschrieben. Geschildert ist hier insbsondere die Herstellung von 11$\alpha$- und/oder $\beta$-Hydroxy-$\Delta$1,4-BNC und 11 Keto-$\Delta$1,4-BNC durch mikrobielle Oxidation der entsprechenden BNC-Verbindungen mit Wasserstoff in 11-Position. Die Herstellung von $\Delta$4,17(20)-BNC-Verbindungen bzw. $\Delta$1,4,17(20)-BNC-Verbindungen mit einer Sauerstoff-Funktion in 11-Stellung — ebenfalls durch mikrobielle Oxidation der entsprechenden BNC-Ausgangsverbindungen mit Wasserstoff in 11-Position — ist Gegenstand der parallelen Schutzrechtsanmeldungen 81 103 423.0 (EP-A-39 894) der Anmelderin (»Neue $\Delta$17(20)-BNC-Verbindungen und Verfahren zu ihrer Herstellung«, Priorität Österreich A 2534/80 vom 12. Mai 1980).

Die vorliegende Erfindung geht von der Aufgabe aus, 11-oxidierte Steroidcarbonsäuren mit einer Carboxylgruppe in 20-Stellung in solche funktionelle Derivate umzuwandeln, die geeignete Ausgangsmaterialien für eine weitere chemische Transformation sein können. Die Erfindung will weiterhin insbesondere neue 20-Steroidcarbonsäurederivate mit einer Sauerstoff-Funktion in 11-Stellung zur Verfügung stellen, die für den angegebenen Zweck geeignet sind.

Gegenstand der vorliegenden Erfindung sind dementsprechend in einer ersten Ausführungsform neue $\Delta$4- und gegebenenfalls weitere Doppelbindungen in 1- und/oder 17-Stellung sowie eine 11-Sauerstoff-Funktion aufweisende Steroidderivate der allgemeinen Formel I

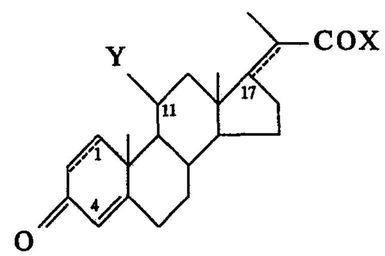

(I)

in der X Halogen oder $NH_2$ und Y eine Hydroxylgruppe oder gemeinsam mit dem durch Y substituierten C-Atom in 11-Stellung eine Carbonylgruppe bedeuten. Die bevorzugten Halogene sind Brom, vor allem aber Chlor. Gegenstand der Erfindung sind weiterhin Verfahren zur Herstellung dieser Verbindungen der allgemeinen Formel I, die noch im einzelnen geschildert werden, sowie die neuen Steroidderivate der allgemeinen Formel I.

Die erfindungsgemäßen neuen Steroid-20-Carbonsäurederivate leiten sich von 4 Grundtypen ab. Hierbei handelt es sich um die — jeweils in 11-Stellung die Sauerstoff-Funktion Y aufweisenden — Steroid-20-Carbonsäuren entsprechend der Δ4-BNC, der Δ1,4-BNC, der Δ1,4,17-BNC und der Δ4,17-BNC. Die Erfindung wird im folgenden eingehend anhand der Δ1,4-BNC und ihrer Derivate geschildert, ist hierauf jedoch nicht eingeschränkt, auch die entsprechenden Δ4-, Δ1,4,17- und Δ4,17-Verbindungen fallen in den erfindungsgemäßen Rahmen.

In einer ersten speziellen Ausführungsform betrifft die Erfindung 11-Hydroxy-pregna-1,4-dien-3-on-20-carbonsäurederivate der allgemeinen Formel II

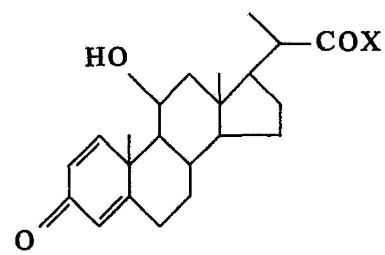

(II)

in der X die angegebene Bedeutung hat, insbesondere also Chlor oder Brom bzw. $NH_2$ bedeutet. Die Hydroxylgruppe in 11-Stellung kann in α- oder in β-Stellung vorliegen. Beide Ausführungsformen fallen in den Rahmen der Erfindung. Aus den eingangs angegebenen Gründen der erhöhten pharmakologischen Wirksamkeit haben Verbindungen der allgemeinen Formel II mit einer β-Hydroxygruppierung wiederum besondere Bedeutung. Die wichtigsten Verbindungen der hier angesprochenen Art sind damit 11β-Hydroxy-pregna-1,4-dien-3-on-20-Carbonsäurechlorid und das entsprechende -20-Carbonsäureamid- bzw. die vergleichbaren Verbindungen der Δ4-BNC, der Δ1,4,17-BNC und der Δ4,17-BNC.

11-α-Hydroxy-Derivate der angegebenen Art können gewünschtenfalls in an sich bekannter Weise in entsprechende 11-β-Hydroxyverbindungen umgewandelt werden, siehe hierzu die eingangs zitierte Literatur des Standes der Technik.

In einer weiteren speziellen Ausführungsform betrifft die Erfindung Steroidderivate der angegebenen Art, in denen Y — zusammen mit dem durch Y substituierten C-Atom — eine Carbonylgruppe bedeutet. Insbesondere betroffen sind hier Pregna-1, 4-dien-3,11-dion-20-carbonsäurederivate der allgemeinen Formel III

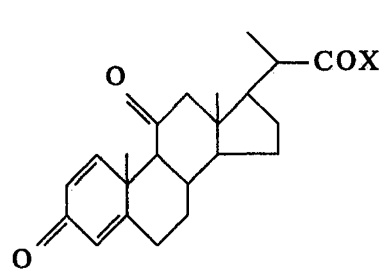

(III)

in der X Halogen, insbesondere Chlor oder Brom, oder $NH_2$ bedeutet. Besonders wichtige Verbindungen sind das Pregna-1,4-dien-3,11-dion-20-carbonsäurechlorid sowie das entsprechende 20-Carbonsäureamid und die vergleichbaren Derivate der Δ4-BNC, Δ1,4,17-BNC und Δ4,17-BNC.

Halogenide bzw. Amide der hier geschilderten Art sind bisher in der Literatur nicht beschrieben. Sie eignen sich in besonderer Weise als Ausgangsmaterial für nachfolgende Reaktionen zur weiteren strukturellen Umwandlung des Seitenkettensubstituenten in 17-Stellung des Steroidringgerüsts.

Die Herstellung der neuen Verbindungen der allgemeinen Formel I sowie die Kennzeichnung wichtiger bestimmter Verbindungen durch analytische Daten sind im einzelnen in den nachfolgenden Beispielen beschrieben. Allgemein gelten jedoch zur Herstellung der neuen Verbindungen die folgenden Angaben:

Die Überführung von Carbonsäuren in Säurehalogenide — insbesondere Säurechloride — unter Verwendung von Halogenierungsmitteln wie Phosphorhalogenide, Oxalylhalogenid oder insbesondere Thionylhalogenid ist eine an sich seit langem bekannte und allgemein benutzte Reaktion, auch in der Reihe der 20-Carboxypregnan-Derivate.

Wendet man aber die in der Literatur — siehe hierzu beispielsweise Fiat Final Report Nr. 996, Seite 24 ff sowie P. L. Julian, E. D. Meyer und H. C. Printy, J. Am. Chem. Soc. 70, 887 (1948) — für die Umsetzung von 3-Acetoxy-bis-norcholensäure mit Thionylchlorid angegebenen Reaktionsbedingungen auf beispielsweise $\Delta$1,4-BNC an, verestert danach das so erhaltene Säurechlorid mit Methanol und analysiert das Rohprodukt, so findet man im Gaschromatogramm einen zusätzlichen Peak und bei der Elementaranalyse einen deutlichen zunächst nicht erwarteten Cl-Gehalt. Ähnliches gilt sogar in noch stärkerem Maße für die Verwendung von Oxalylchlorid anstelle von Thionylchlorid.

Der Grund für das Auftreten dieser unerwünschten Verunreinigungen, die die Ausbeute an gewünschtem Produkt deutlich senken und zu Reinigungsproblemen bei weiteren Umsetzungen mit dem Säurechlorid führen können, ist wahrscheinlich eine Chlorierung mit eventuell anschließender Aromatisierung des A-Ringes im Steroidgerüst, wie sie z. B. für die Umsetzung von Androsta-1,4-dien-3,17-dion(ADD) mit Oxalylchlorid bekannt ist; siehe hierzu G. B. Moersch et al., J. Org. Chemistry, 29, 2495 (1964).

Überraschenderweise gelingt die Bildung der erfindungsgemäß gewünschten Säurehalogenide bereits unter außergewöhnlich milden Reaktionsbedingungen, bei denen die unerwünschte Mitreaktion anderer reaktiver Stellen der zugrunde liegenden ein- oder mehrfach ungesättigten BNC-Struktur noch nicht stattfindet. So zeigte sich das überraschende Ergebnis, daß beispielsweise eine praktisch quantitative Säurechloridbildung stattfindet, wenn die folgenden Reaktionsbedingungen eingehalten werden: Reaktionstemperaturen unter 15°C, vorzugsweise unter 5°C, insbesondere im Bereich von 0 bis 5°C, stöchiometrische Mengen der Reaktanten oder nur sehr begrenzter Überschuß des Halogenierungsmittels, der vorzugsweise nicht über 20 Mol-% und insbesondere nicht über 10 Mol-% beträgt, sowie Arbeiten in Gegenwart eines inerten Verdünnungsmittels, gewünschtenfalls in Anwesenheit geringer Mengen eines basischen Katalysators.

Als inerte Lösungsmittel kommen beispielsweise halogenierte Kohlenwasserstoffe oder, mit Einschränkungen, Ether in Betracht. Geeignete inerte Lösungsmittel sind beispielsweise Methylenchlorid oder Chloroform. Als Halogenierungsmittel kommen Phosphorhalogenide, insbesondere $PCl_3$ oder $PCl_5$ bzw. die entsprechenden Bromide, vor allen Dingen jedoch das Thionylhalogenid, insbesondere Thionylchlorid, in Betracht. Katalytische Mengen einer Base, insbesondere Pyridin oder Dimethylformamid beschleunigen die Reaktion, sind aber häufig nicht erforderlich. In Einzelfällen kann die Mitverwendung eines Katalysators wünschenswert sein , ein Beispiel hierfür ist die Herstellung des Säurechlorids der $\Delta$1,4-11$\beta$-OH-BNC. Pyridin wird als Katalysator bevorzugt.

Je nach Wahl der sonstigen Verfahrensparameter bzw. der miteinander umzusetzenden Verbindungen können einzelne Verfahrensparameter auch außerhalb der bisher angegebenen Werte liegen. So kann beispielsweise die Verfahrenstemperatur im Bereich von etwa —20°C bis etwa 75°C liegen, vorausgesetzt, daß bei den höheren Temperaturen des genannten Bereiches durch geeignete sonstige Verfahrensführung das Entstehen unerwünschter Ringhalogenierungsprodukte vermieden wird.

Die Menge des Halogenierungsmittels kann — wiederum geeignete Abstimmung der sonstigen Verfahrensbedingungen vorausgesetzt — auch in nicht unbeträchtlichem Überschuß über die stöchiometrisch benötigte Menge hinaus vorliegen. So sind beispielsweise Mengen bis zu fünf Äquivalenten, vorzugsweise bis zu drei Äquivalenten des Halogenierungsmittels in Sonderfällen brauchbar. Die Reaktion wird üblicherweise bei Normaldruck durchgeführt. Das Halogenierungsmittel wird zweckmäßigerweise zu der Lösung der umzusetzenden Steroidverbindung im inerten Lösungsmittel gegeben. Es hat sich als vorteilhaft erwiesen, das Halogenierungsmittel in möglichst reiner Form einzusetzen. Offenbar fördern üblicherweise im Halogenierungsmittel vorliegende Verunreinigungen unerwünschte Nebenreaktionen. Zweckmäßig ist beispielsweise eine Reinigung des Halogenierungsmittels mit einer ungesättigten Verbindung wie Leinöl oder besonders Squalen. Diese ungesättigten Komponenten reagieren mit den Verunreinigungen im Halogenierungsmittel und senken damit die Bildung unerwünschter Nebenprodukte auf ein Minimum.

Aus den Säurehalogeniden können die erfindungsgemäßen neuen Carbonsäureamide der allgemeinen Formel I gewonnen werden, d. h. Verbindungen dieser Art, in denen X die $NH_2$-Gruppe bedeutet. Zu ihrer Herstellung bringt man das jeweils zugehörige 20-Carbonsäurehalogenid mit Ammoniak oder einer Ammoniak liefernden Verbindung zur Umsetzung. Diese Umsetzung wird zweckmäßigerweise im Temperaturbereich von etwa —20°C bis etwa 80°C, vorzugsweise im Bereich von etwa —5°C bis etwa 35°C, durchgeführt. Ammoniak bzw. die unter den Reaktionsbedingungen Ammoniak liefernde

Verbindung wird wenigstens in etwa equimolaren Verhältnissen eingesetzt. Geeignet sind beispielsweise Mengen des Ammoniaks bzw. der Ammoniak liefernden Verbindung von 1,1 bis 5 Äquivalenten — bezogen auf Säurehalogenid — vorzugsweise Mengen von etwa 1,2 bis etwa 3 Äquivalenten. Wird nicht Ammoniak selber, sondern eine Ammoniak liefernde Verbindung eingesetzt, so eignet sich hierfür insbesondere Ammoniumhydroxid.

Die Umsetzung des 20-Carbonsäurehalogenids mit Ammoniak bzw. der Ammoniak liefernden Verbindung erfolgt vorzugsweise ebenfalls in einem organischen Lösungsmittel, beispielsweise in Halogenkohlenwasserstoffen, wie zuvor angegeben. Ein besonders geeignetes inertes Lösungsmittel ist auch hier Methylenchlorid oder Chloroform.

Wird Ammoniumhydroxid als Ammoniak liefernde Komponente eingesetzt, so fällt im Reaktionsgemisch neben der organischen Phase eine wäßrige Phase an. Durch einfache Phasentrennung oder auch durch Abtrennung des fest ausgefallenen Amids gelingt die Gewinnung des Reaktionsproduktes. Die abgetrennte organische Phase wird zweckmäßigerweise mehrfach mit Wasser gewaschen, anschließend mit beispielsweise Calciumsulfat getrocknet und gefiltert. Das als inertes Verdünnungsmittel eingesetzte organische Lösungsmittel wird abgetrennt, die Carboxamidoverbindung kann dann in an sich bekannter Weise weiter gereinigt werden.

Bei der Umsetzung zwischen Carbonsäurehalogenid und Ammoniak freiwerdende Halogenwasserstoffsäure wird entweder durch einen Überschuß von Ammoniak bzw. Ammoniumhydroxid gebunden, es kann aber auch eine basische Komponente zur Bindung der freiwerdenden Säure mitverwendet werden.

Die Gewinnung der 11-Oxoverbindungen ist auch durch Oxidation der entsprechenden 11-Hydroyverbindungen möglich. Hier ist also neben dem unmittelbaren Zugang über 11-Oxo-Ausgangsmaterialien auch der Zugang über 11-Hydroxyausgangsverbindungen gegeben.

Sowohl die zunächst beschriebenen Steroid-20-Carbonsäurehalogenide — insbesondere die entsprechenden Chloride — als auch die daraus gewonnenen 20-Carbonsäureamide sind wertvolle Ausgangsprodukte für eine weitergehende Umwandlung des Substituenten in 17-C-Stellung.

Beispiel 1

Pregna-1,4-dien-3,11-dion-20-carbonylchlorid

500 mg (1,4 mmol) Δ1,4-11-Oxo-BNC in 8 ml trockenem $CH_2Cl_2$ werden bei 0° C mit 0,12 ml (1,6 mmol) frisch über Squalen destilliertem Thionylchlorid und einem kleinen Tropfen Pyridin versetzt. Nach 1 h wurden das Lösungsmittel und überschüssiges Thionylchlorid bei 0° C im Vakuum entfernt. Der Rückstand wird mit 5 ml abs. Methylenchlorid versetzt und nochmals zur Trockne eingeengt. Die Auswaage des rohen Säurechlorids beträgt 495 mg.

Zur Überprüfung der Ausbeute wurde das rohe Säurechlorid in 10 ml abs. Methylenchlorid gelöst und bei 0° C mit 2 ml trockenem Methanol und 0,5 ml Pyridin versetzt. Die quantitative DC-Analyse nach Überführung des gebildeten Esters in einen Meßzylinder ergab 470 mg (90%) des Methylesters.

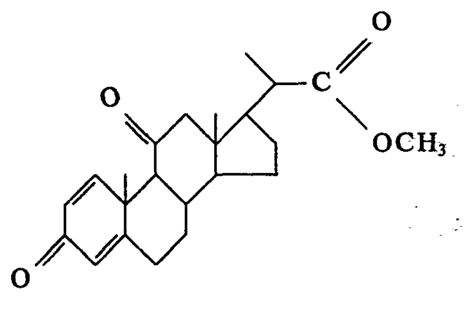

Beispiel 2

11 β-Hydroxy-pregna-1,4-dien-3-on-20-carbonylchlorid

Die Darstellung wurde analog Beispiel 1 durchgeführt. Zur Überprüfung der Ausbeute wurde das Reaktionsprodukt in den Methylester überführt.
Ausbeute: 88%

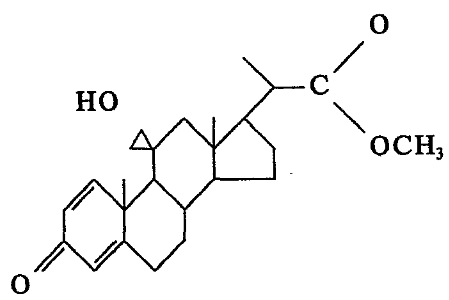

Mit Beispiel 1 und Beispiel 2 vergleichbare Ergebnisse werden bei der Verwendung von Δ4-BNC, Δ1,4,17-BNC, Δ4,17-BNC oder Mischungen von 2 oder mehreren der hier genannten Komponenten erhalten.

## Beispiel 3

### Pregna-1,4-dien-3,11-dion-20-carboxamid

Aus 1 g $\Delta^{1,4}$-11-Oxo-BNC wurde gemäß Beispiel 1 das Säurechlorid hergestellt.

In dessen Lösung in 10 ml trockenem Methylenchlorid wurde 5 min lang trockenes $NH_3$ eingeleitet, wobei ein weißer Niederschlag ausfiel. Die Reaktionsmischung wurde mit etwas $CH_2Cl_2$ verdünnt, mit verdünnter HCl, $NaHCO_3$-Lösung und anschließend mit Wasser gewaschen. Nach dem Trocknen der $CH_2Cl_2$-Phase über $Na_2SO_4$ wurde zur Trockne eingeengt und der Rückstand mit Ether verrührt. Nach dem Filtrieren verblieben 880 mg weißer, kristalliner Rückstand, der nach DC (Kieselgel; $CH_2Cl_2$/Essigester/$CH_3OH$ 5 : 4 : 1 + 2% Triethylamin) nur unwesentlich verunreinigt war.

Fp (aus n-Butanol) 220—225° C (Zers.)

$C_{22}H_{29}NO_3$
gef.:C 74,08;H 8,04;N 3,78%
ber.:C 74,33,H 8,22;N 3,94%

## Beispiel 4

### 11 β-Hydroxy-pregna-1,4-dien-3-on-20-carboxamid

1 g $\Delta^{1,4}$-11-β-OH-BNC wurde wie in Beispiel 2 beschrieben in das Amid übergeführt.

Nach Beendigung des $NH_3$-Einleitens wurde mit $CH_2Cl_2$ verdünnt und zunächst mit kalter 2 n HCl versetzt. Dabei fiel das Amid zum Teil als gelb gefärbter, schmieriger Feststoff aus. Dieser wurde abgetrennt und in heißem n-Butanol gelöst. Beim Stehenlassen kristallisierte nach längerer Zeit das Amid in weißen Kristallen aus.

Insgesamt wurden ca. 820 mg des Amids erhalten (Reinfraktion sowie aus den Lösungen durch Einengen erhaltenen Mengen).

Fp (aus n-Butanol): 260° C (Zers.)

$C_{22}H_{31}NO_3$
gef.:C 74,13;H 8,89,N 3,79%
ber.:C 73,91;H 8,74;N 3,92%

**Patentansprüche**

1. Δ4- und gegebenenfalls weitere Doppelbindungen in 1- und/oder 17-Stellung sowie eine 11-Sauerstoff-Funktion aufweisende Steroidverbindungen der allgemeinen Formel I

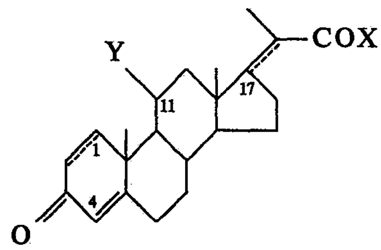

(I)

in der X Halogen, insbesondere Chlor oder Brom, oder $NH_2$ und Y eine Hydroxylgruppe oder gemeinsam mit dem durch Y substituierten C-Atom eine Carbonylgruppe bedeuten.

2. 11-Hydroxy-pregna-1,4-dien-3-on-20-carbonsäurederivate der allgemeinen Formel II

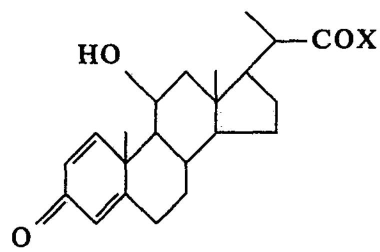

(II)

in der X Halogen, insbesondere Chlor oder Brom, oder $NH_2$ bedeutet.

3. 11β-Hydroxy-pregna-1,4-dien-3-on-20-carbonsäurederivate der allgemeinen Formel II, in der X die in Anspruch 2 angegebene Bedeutung hat.

4. 11β-Hydroxy-pregna-1,4-dien-3-on-20-carbonsäurechlorid.

5. 11β-Hydroxy-pregna-1,4-dien-3-on-20-carbonsäureamid.

6. Pregna-1,4-dien-3,11-dion-20-carbonsäurederivate der allgemeinen Formel III

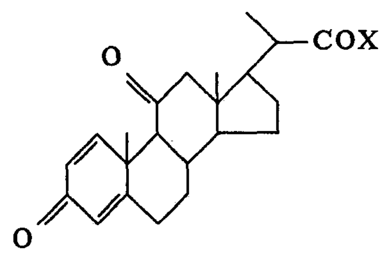

(III)

in der X Halogen, insbesondere Chlor oder Brom oder $NH_2$ bedeutet.

7. Pregna-1,4-dien-3,11-dion-20-carbonsäurechlorid.

8. Pregna-1,4-dien-3,11-dion-20-carbonsäureamid.

9. Verfahren zur Herstellung von Steroid-20-Carbonsäureverbindungen der allgemeinen Formel I, in der X Halogen, insbesondere Chlor oder Brom oder $-NH_2$ und Y eine Hydroxylgruppe oder gemeinsam mit dem durch Y substituierten C-Atom eine Carbonylgruppe bedeuten, dadurch gekennzeichnet, daß man die zugehörige Steroid-20-Carbonsäure mit einem Halogenierungsmittel in annähernd stöchiometrischen Mengen gegebenenfalls in Gegenwart eines basischen Katalysators bei Temperaturen nicht über 15° C, vorzugsweise unter 5° C, umsetzt und gewünschtenfalls das gebildete Steroid-20-Carbonsäurehalogenid mit Ammoniak oder einer Ammoniak liefernden Verbindung zur Reaktion bringt.

10. Verfahren nach Anspruch 9, dadurch gekennzeichnet, daß man ein anorganisches Halogenierungsmittel, z. B. Phosphor-tri- bzw. -pentahalogenid, insbesondere aber Thionylhalogenid, einsetzt.

11. Verfahren nach Ansprüchen 9 und 10, dadurch gekennzeichnet, daß man in Gegenwart eines inerten Lösungsmittels, wie halogenierten Kohlenwasserstoffen, arbeitet.

**Claims**

1. Δ4-steroid compounds optionally containing other double bonds in the 1- and/or 17-position and also an 11-oxygen function and corresponding to the following general formula

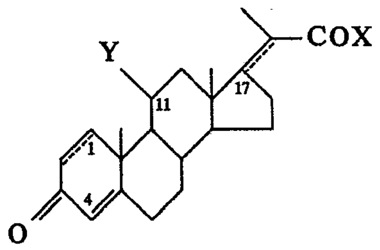

(I)

in which X represents halogen, particularly chlorine or bromine, or $NH_2$ and Y is a hydroxyl group or, together with the C-atom substituted by Y, represents a carbonyl group.

2. 11-hydroxy-pregna-1,4-dien-3-one-20-carboxylic acid derivatives corresponding to the following general formula

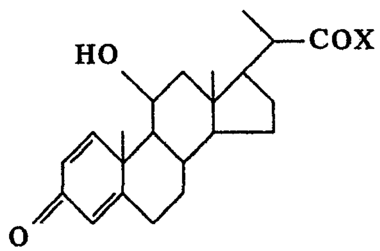

(II)

in which X represents halogen, particularly chlorine or bromine, or $NH_2$.

3. 11β-hydroxy-pregna-1,4-dien-3-one-20-carboxylic acid derivatives corresponding to general formula II, in which X is as defined in claim 2.

4. 11β-hydroxy-pregna-1,4-dien-3-one-20-carboxylic acid chloride.

5. 11β-hydroxy-pregna-1,4-dien-3-one-20-carboxylic acid amide.

6. Pregna-1,4-dien-3-11-dione-20-carboxylic acid derivatives corresponding to the following general formula

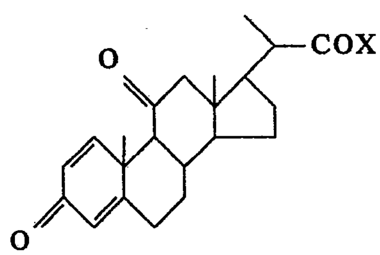

(III)

in which X represents halogen, particularly chlorine or bromine, or $NH_2$.

7. Pregna-1,4-dien-3,11-dione-20-carboxylic acid chloride.

8. Pregna-1,4,-dien-3,11-dione-20-carboxylic acid amide.

9. A process for producing steroid-20-carboxylic acid compounds corresponding to general formula I, in which X represents halogen, particularly chlorine or bromine, or $-NH_2$ and Y is a hydroxyl group or, together with the C-atom substituted by Y, represents a carbonyl group, characterized in that the associated steroid-20-carboxylic acid is reacted with a halogenating agent in substantially stoichiometric quantities, optionally in the presence of a basic catalyst, at temperatures not exceeding 15°C and preferably below 5°C, if desired, the steroid-20-carboxylic acid halide formed is reacted with ammonia or an ammonia-yielding compound.

10. A process as claimed in claim 9, characterized in that an inorganic halogenating agent, for example phosphorus trihalide oı pentahalide, but especially thionyl halide, is used.

11. A process as claimed in claims 9 and 10, characterized in that the reaction is carried out in the presence of an inert solvent, such as a halogenated hydrocarbon.

**Revendications**

1. Composés stéroïdiques portant une double liaison en $\Delta 4$ et le cas échéant d'autres doubles liaisons en positions 1 et/ou 17 ainsi qu'une fonction oxygénée en position 11, de formule générale I

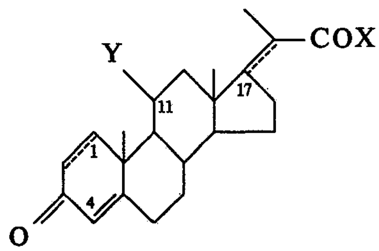

(I)

dans laquelle X représente un halogène, en particulier le chlore ou le brome, ou un groupe $NH_2$ et Y représente un groupe hydroxy ou forme un groupe carbonyle avec l'atome de carbone substitué par Y.

2. Dérivés de l'acide 11-hydroxy-prégna-1,4-diène-3-one-20-carboxylique de formule générale II

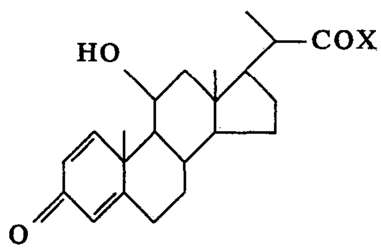

(II)

dans laquelle X représente un halogène, en particulier le chlore ou le brome, ou un groupe $NH_2$.

3. Dérivés de l'acide 11 bêta-hydroxy-prégna-1,4-diène-3-one-20-carboxylique de formule générale II dans laquelle X a la signification indiquée dans la revendication 2.

4. Le chlorure de l'acide 11 bêta-hydroxy-prégna-1,4-diène-3-one-20-carboxylique.

5. L'amide de l'acide 11 bêta-hydroxy-prégna-1,4-diène-3-one-20-carboxylique.

6. Dérivés de l'acide prégna-1,4-diène-3,11-dione-20-carboxylique de formule générale III

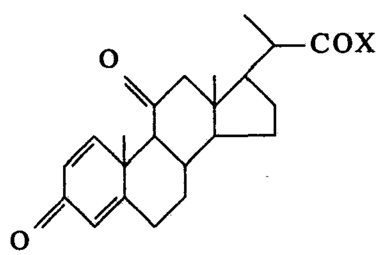

(III)

dans laquelle X représente un halogène, en particulier le chlore ou le brome, ou le groupe $NH_2$.

7. Le chlorure de l'acide prégna-1,4-diène-3,11-dione-20-carboxylique.

8. L'amide de l'acide prégna-1,4-diène-3,11-dione-20-carboxylique.

9. Procédé de préparation des dérivés d'acides stéroïde-20-carboxyliques de formule générale I dans laquelle X représente un halogène, en particulier le chlore ou le brome, ou le groupe $-NH_2$, et Y représente un groupe hydroxy ou forme un groupe carbonyle avec l'atome de carbone substitué par Y, caractérisé en ce que l'on fait réagir l'acide stéroïde-20-carboxylique correspondant avec un agent halogénant en proportions à peu près stoechiométriques, éventuellement en présence d'un catalyseur basique, à des températures ne dépassant pas 15° C, de préférence à des températures inférieures à 5° C, et si on le désire, on fait réagir l'halogénure d'acide stéroïde-20-carboxylique formé avec l'ammoniac ou un composé libérant de l'ammoniac.

10. Procédé selon la revendication 9, caractérisé en ce que l'on utilise un agent halogénant minéral, par exemple un tri- ou penta-halogénure de phosphore et plus spécialement un halogénure de thionyle.

11. Procédé selon les revendications 9 et 10, caractérisé en ce que l'on opère en présence d'un solvant inerte tel qu'un hydrocarbure halogéné.